# EUROPEAN PATENT APPLICATION

(11) **EP 2 765 185 A2**
(43) Date of publication of application: **13.08.2014**
(21) Application number: 13464002.8
(22) Date of filing: 13.02.2013
(51) Int. Cl.: C12N 1/20, A01N 63/00, C12R 1/07

(54) **Strain of Brevibacillus parabrevis and controlled release composition based on it**

(30) Priority: 11.02.2013 RO 201300141
(71) Applicant: SC Euro Bio SRL, 075100 Otopeni, jud. Ilfov (RO)
(72) Inventor: Oancea, Florin, 062082 Bucharest (RO); Pairault, Adriana-Liliana, 077190 Voluntari jud. Ilfov (RO); Doni, Mihaela, 031747 Bucharest (RO); Raut, Iuliana, 032768 Bucharest (RO); Calin, Mariana, 060962 Bucharest (RO); Jecu, Maria-Luiza, 060144 Bucharest (RO)

(57) **Abstract**

Invention relates to a strain of *Brevibacillus parabrevis* B50, NCAIM (P) B 001413, which present superior characteristics of: *in vitro* antagonism against phytopathogen agents; biosynthesis of compounds which stimulate the formation of mycorrhiza; endophyte development; innocuity on *Galleria mellonella* test; protection of maize plants, used as host plants for the multiplication of mycorrhizal fungi, and ornamental plants of geranium, pansies, petunias and cockscomb, against soil phytopathogen agents; stimulation of mycorrhiza on the host plants used for multiplication of mycorrhizal fungi, with the formation of suppressive mycorrhizal composts. The controlled-release composition based on strain B50 is made up of 76 parts oatmeal flour, 3 parts ethyl esters of fatty acids, 4 parts polyvinyl alcohol, 3.5 parts lecithin, 0.75 parts potassium soap, 0,45 parts glycerol, 0,4 parts fats from rapeseed oil, the balance to 100 parts being water, and min. 10⁸ cfu/g *B. parabrevis* B50

## Description

This invention relates to a strain of *Brevibacillus parabrevis,* showing antagonism to phytopathogen agents from soil and capacity to promote the development of mycorrhizal symbiosis on plant roots, and to a controlled-release composition containing this strain, intended to be used for the formation of the complex suppressive composts, which include also mycorrhizal spores, composts useful for plant protection and nutrition, especially of ornamentals plants.

There are of known a number of strains of Gram-positive endo-sporing bacteria, belonging to the genus *Brevibacillus,* which are intended for use in agriculture. Patent RU2422511 (C1) describes the strain 16-336 of *Brevibacillus laterosporus,* deposited under the number B-10531 in the Russian National Collection of Industrial Microorganisms, which produces a wide spectrum of biologically active compounds, inhibiting growth of harmful algae and microscopic phytopathogenic fungi. Patent application WO2010142055 (A3) refers to a composition with a broad spectrum of activity against phytopathogenic bacteria and microscopic fungi producing late blight, done on the basis of a consortium of Gram-positive endo-sporing bacteria, which include also strains of *Brevibacillus parabrevis.* US patent 5055293 (A) protects the strain P5 *Brevibacillus laterosporus* (deposit number ATCC 53694), which is pathogenic to a whole series of lepidopteran crop pest, and especially to Western Corn Rootworm *(Diabrotica virgifera virgifera*)*.* Strain P5 is applied as soil treatment or seed treatment, to limit harmful lepidopteran populations toward it is active. Patent application RO 127467 (A2) presents a new strain of *Brevibacillus laterosporus,* 56.1 s, deposit number DSM 23663, which present, in the same time, antagonism against phytopathogenic fungi from soil and high capacity of colonization of plant material and plant tissues, and which is intended for use in farms with conservation farming systems.

There have not yet described strains from *Brevibacillus* genera having, simultaneous, activity of antagonism towards soil phytopathogen agents and ability to promote the development of mycorrhizal symbiosis on plant roots. Such strains should allow the reproducible obtainment of suppressive composts with a high content of mycorrhizal spores, stimulating the formation of mycorrhiza on host plants on which are multiplied the mycorrhiza biotrophic fungi, and eliminating the need for application of chemical products, for protection of multiplying plants against attack of soil phytopathogens. Patent CN101914469 (B) claim the HB12 strain of *Bacillus cereus,* stored as CCTCC No. M 209025, which has the capacity to stimulate development of ecto-mycorrhiza. Besides the fact that for this strain of gram-positive endo-sporing bacteria were not described also antagonism characteristics against phytopathogens, another disadvantage is the significant health risk, strains of *Bacillus cereus* being enterotoxigenic (The EFSA Journal, 2005, 172: 1-48). The strains which are used for protection and nutrition of ornamental crops from the green spaces (including through endo-mycorrhiza development stimulation), shall not present pathogenicity to humans or non-target organisms, and this innocuity should be tested since the screening stage.

The technical problem which the invention intends to solve is to present a strain, isolated from natural habitats, which is not pathogenic to humans and non-target organisms, and which presents, in the same time, characteristics of: antagonism for a number of phytopathogen agents and stimulation of the development of endo-mycorrhiza on plant roots. For such strains is also required a controlled release compositions, which should facilitate the colonization of substrates with said microorganism, assuring the formation of suppressive and complex composts containing mycorrhizal spores. Such composition must protect the bacteria spores, remained non-released, against bacteriostatic action of bacteriocins produced by vegetative forms of the same species which have colonized already the substrate. The antagonism toward phytopathogen agents of the Gram-positive endo-sporing strains is due mainly to bacteriocins, with a lipopeptidic structure (Ongena and Jacques, 2008, Trends Microbiol. 16:115-125), and these bacteriocins have as main ecological role limitation of competing micro-organisms development, including of competitors within the same species (Riley and Wertz, 2002. Annu. Rev. Microbiol. 56: 117-137).

This invention relates to the strain *Brevibacillus parabrevis* B50, deposited with the deposit number NCAIM (P) B 001413 in National Collection of Agricultural and Industrial Microorganisms, Corvinus University of Budapest. The strain of *Brevibacillus parabrevis* B50 was selected from more than 125 isolates from soil, showing superior characteristics for:
✔ *In vitro* antagonism against phytopathogen agents, *Fusarium graminearum, Sclerotinia sclerotiorum, Alternaria spp., Rhizoctonia solani, Fusarium oxysporum* f. sp. *dianthi, Sclerotium bataticola, Botrytis cinerea ;*
✔ Biosynthesis of compounds which stimulate the formation of mycorrhiza (manganese dioxide, polyamines) and endophyte development;
✔ Innocuity on *Galleria mellonella* test;
✔ Protection of maize plants, used as host plants for the multiplication of mycorrhizal fungi, and of ornamental plants of geranium, pansies, petunias and cockscomb, against soil phytopathogen agents;
✔ Stimulation of mycorrhiza on the host plants used for multiplication of mycorrhizal fungi, with the formation of suppressive mycorrhizal composts.

The controlled-release formulation based on strain *Brevibacillus parabrevis* B50 is made up of 76 parts oatmeal flour, 3 parts ethyl esters of fatty acids, 4 parts polyvinyl alcohol, 3.5 parts lecithin, 0.75 parts potassium soap, 0.45 parts glycerol, 0.4 parts unsaponifiable fats from rapeseed oil, the balance to 100 parts being water, and min. 10⁸ cfu/g *B. parabrevis* B50.

By applying the invention are obtained the following advantages:
- stimulation and protection of maize plants used for multiplication of mycorrhizal fungi, excluding chemicals products which may interfere with the formation of mycorrhiza;
- possibility to obtain, in a reproducible manner, suppressive composts with a high content of mycorrhizal spores, intended for an integrated biological plant nutrition and protection, especially on ornamentals plant;
- Assurance of an uniform and reproducible colonization by the strain B50 of the mychorriza plant growing substrate, in the case of controlled-released composition application, remaining spores, not-released initially from the composition according to this invention, being protected from the bacteriostatic action of lipopeptides with bacteriocinic activity, synthesized by vegetative forms of the same strain developed from the first wave released, and which already colonized the substrate, due to preponderant accumulation of bacteriocins lipopeptides in the hydrophobic part of the composition;
- Eliminate risks which could result from the use of bacterial strains that stimulates the formation of mychorriza, but are potentially pathogenic to humans and other non-target organisms.

Further the invention will be described in detail in the following examples.

*Example 1.* Strain the B50 *Brevibacillus parabrevis* was obtained on the National Institute of Research-Development for Chemistry and Petrochemistry, ICECHIM Bucharest, from a sample of soil from Southern Muntenia. For the isolation of the bacteria was used nutrient agar (peptone-5 g/l, meat extract - 3 g/l agar 20 g/l, on 1000 ml water pH 6.8-7.2), and for the cultivation was used agarized Luria-Bertani medium (LBA: bactotripton 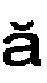 -10 g/l yeast extract 5 g/l NaCl-10 g/l agar 20 g/l, 1000 ml water, pH 7.5), on an optimum incubation temperature of 28°C.

This strain was selected from a collection of more than 125 isolates gram-negative sporing bacilli, based on laboratory tests through which were determined the following:
✔ antagonistic activity *in vitro* against phytopathogenic fungi *(Fusarium graminearum, Sclerotinia sclerotiorum, Alternaria spp., Rhizoctonia solani, Fusarium oxysporum* f. sp. *dianthi, Sclerotium bataticola, Botrytis cinerea);*
✔ production of amylase and AHL lactonase;
✔ migration mobility / *swimming* and aggregation / *swarming;*
✔ biofilm formation *in vitro*;
✔ biosynthesis of compounds which stimulate the formation of mychorriza (manganese dioxide, polyamines);
✔ Innocuity on *Galleria mellonella* test;
✔ Protection activity of corn seedlings against the attack of *Fusarium graminearum,* with endophytic development of the bacterium B50.

For taxonomic classification, B50 strain was characterized on the basis of a polyphasic taxonomy, i.e. a combination of morphological characters of cells and colonies on different growing media (table 1), with the physiological (table 2) and molecular (sequence 16S rADN, profile of fatty acids from membrane lipids, tab 3) ones. Corroborating all these data, the strain B50 was classified as a belonging to the species *Brevibacillus parabrevis.*

**Tab. 1. The morphology of the cells and colonies of Brevibacillus parabrevis B50 after cultivation for 24 hours.**

| | |
|---|---|
| Cells: | *form*: rod |
| | *dimensions:* 0.9 - 1.0 x 3.0--> 5 µm |
| | *flagella arrangement:* peritrichous flagella |
| Colony - potato-dextrose agar medium | *form:* circular |
| | *external appearance:* rough, irregular edges in the form of filaments |
| | *color:* brown |
| | *dimensions:* average |
| Colony - meat extract (beef extract) medium | *form:* circular |
| | *external appearance:* rough, prominent center, crinkled, irregular edges |
| | *color:* cream-beige |
| | *dimensions:* average |
| Colony - bean extract medium | *form:* circular |
| | *external appearance*: rough, irregular edges |
| | *color:* cream-brown |
| | *dimensions:* average |
| Colony - soil extract medium | *form:* circular |
| | *external appearance*: rough, irregular edges |
| | *color:* cream-brown |
| | *dimensions:* average |

**Tab. 2. Physiological characteristics of strain Brevibacillus parabrevis B50.**

| | |
|---|---|
| Biochemical test | B50 |
| Gram Reaction | ± |
| Voges-Proskauer Reaction | - |
| Starch hydrolysis | + |
| Gelatin hydrolysis | + |
| Hydrolysis Tween 60 | + |
| Reduction NO₃→NO₂ | + |
| Anaerobic growth | - |
| Carbon source: | + |
| malonate | + |
| citrate | - |
| propionate | + |
| tartrate | + |
| trehaloza | + |
| glucose | + |
| Acidification: | |
| xylose | - |
| glucose | + |
| arabinose | - |
| mannitol | + |
| fructose | - |
| cellobiose | ± |
| Tolerance to NaCl 7% | - |

The identification on the basis of 16S rADN sequence was achieved by using a working protocol characterized by the following steps: obtaining of pure cultures - isolated colonies, inoculation techniques by loop streaking; extraction of bacterial DNA; gel electrophoresis for DNA detection; amplification of 16S rADN sequence through PCR technique and gel electrophoresis; purification of ribozomal DNA; enzymatic amplification of nucleic acids before sequencing; precipitation and drying of DNA coding for 16sRNA. Nucleotide sequencing was carried out with the method Dye Terminator Cycle Sequencing (Perkin Elmer, 1998), using an automated sequencer ABI PRISM 310 (Perkin Elmer). Sequences were analyzed using *CHROMAS 2.33* (Technelysium Pty Ltd). Comparison of the sequences of 16S rADN obtained, with existing sequences in the Gene Bank of NCBI (National Center for Biotechnology Information), was achieved with *BLAST* (Basic Local Alignment Search Tool). The results demonstrated a 99% similarity with the strain IFO 12334 of *Brevibacillus parabrevis.*

Membrane fatty acids profile was determined with the help of a Sherlock^{®} Microbial ID system, using the Instant FAME™ procedure. Esters of fatty acids from bacterial membrane were separated on a gas cromatograf GC 6890N (Agilent Technologies) equipped with classical split/splitless injector, capillary column type Ultra 2 (product code 19091 B -102, with a length of 25 m, internal diameter of 0.2 mm, 5% phenyl methyl siloxan stationary phase, thickness of 0.33 µm) and flame ionization detector (FID). Software used for the acquisition and processing of data were: GC chromatography ChemStation version B.01.03 [204]/2005, and Sherlock Microbial Identification System, version 6.1/2008. There were used chromatographic methods developed and validated by the manufacturer (MIDI, Inc., Newark, DE, USA), using the libraries of profiles of fatty acid methyl esters for aerobic microorganisms from the environment (RTSBA6, version 6.0/2008). As reference material was used a standard mixture of methyl esters of fatty acids with linear-chain, with the number of carbon atoms in the molecule between 9 and 20 (Sherlock rapid and sensitive chromatographic methods for quantitative calibration). Standard microorganisms for verification of microbiological and chemical testing protocols of samples processing were *Bacillus subtilis* (strain ATCC 6633) and *Stenotrophomonas maltophilia* (strain ATCC 13637).

**Tab. 3. Profile of fatty acids in the membrane of strain B50.**

| Retention time | The membrane fatty acid type | Proportion (%) |
|---|---|---|
| 1,8769 | 13: 0 iso | 0,97 |
| 1,9018 | 13: 0 anteiso | 0.41 |
| 2,1668 | 14: 0 iso | 3,29 |
| 2,2777 | 14: 0 | 2.32 |
| 2,4762 | 15: 0 iso | 22,58 |
| 2,5055 | 15: 0 anteiso | 58.55 |
| 2,7257 | w7c alcohol 16: 1 | 0.41 |
| 2,7959 | 16: 0 iso | 2.29 |
| 2,8441 | 16: 1 w11c | 1.02 |
| 2,9135 | 16: 0 | 2.02 |
| 2,9972 | 15: 0 20H | 0.71 |
| 3,0475 | 17: 1 iso w10c | 0.58 |
| 3,1184 | 17: 0 iso | 1.57 |
| 3,1493 | 17: 0 anteiso | 2.93 |
| 3,4887 | 18: 1 w9c | 0,35 |

Antagonistic activity of B50 strain against various phytopathogenic fungi was determined *in vitro* by using the technique of double culture, by streaking agarized media with bacterial biomass within 2 cm from a calibrated disc (5 mm) of mycelium. Phytopathogenic fungi cultures was refreshed on the PDA medium (potato, glucose agar) and incubated at 28°C for 5 days. B50 strain was renewed on agarized Luria-Bertani medium (LBA), by incubation at 28°C for 24 hours. Testing the antagonistic activity *in vitro* was performed on the potato-glucose-agar (PDA) medium. Petri plates with test microorganisms were incubated at 28°C and analyzed for inhibition zone (mm) produced by *B. parabrevis* B50, at 24, 48 and 72 hours. Experiments to test *in vitro* antagonism were repeated three times.

**Tab. 4. In vitro testing of the antagonistic activity of the strain Brevibacillus parabrevis B50 on growth of the mycelia of phytopathogenic fungi (inhibition zone at 72 h, mm).**

| Phytopathogens fungus | Zone of inhibition (mm) induced by strain B50 |
|---|---|
| *Fusarium graminearum* DSM4527 | 5 |
| *Fusarium oxysporum* f. SP. *dianthi* ATCC 204230 | 5 |
| *Botrytis cinerea* DSM 5144 | 7 |
| *Rhizoctonia solani* ATCC 66873 | 6 |
| *Sclerotinia sclerotiorum* DSM 1946 | 7 |
| *Sclerotium bataticola* ATCC 12265 | 8 |
| *Alternaria* spp. | 5 |

Results (tab. 4) demonstrated that B50 strain produces antifungal metabolites which inhibited the development of fungi used into this study. The largest inhibition area was registered against the fungus *Sclerotium bataticola* ATCC 12265 (8 mm), followed by those against *Botrytis cinerea* DSM 5144 and *Sclerotinia sclerotiorum* DSM 1946 (7 mm).

The strain B50 was analyzed for to the production of enzymes that have an important role in the colonization of plant surfaces, in antagonistic activity and modulation of consortiums with various kinds of other micro-organisms.

Production of amylase was analyzed by seeding as streak on the nutrient agar (NA) + 0.4% soluble starch. The plates were incubated at 28°C for 48 to 72 hours, after that were treated with the solution of iodine in potassium iodide through flooding. Clear areas around bacterial colonies formed after adding the solution of iodine in potassium iodide demonstrate the decomposition of starch from the medium and, consequently, the production of amylase. The results shown that the strain B50 produces amylase.

Production of lactonase (enzyme involved in *quorum quencing,* which inhibit communication between the deleterious bacteria using the *quorum sensing* type AHL) was analyzed by inoculation of strain B50 in 2 ml of liquid medium Luria Bertani, on which was added C6-hexanoil homoserin lactone (C6-HHL), in 5 µM final concentration, followed by overnight incubation, at 28°C and 150 rpm. Simultaneously, the same medium, but only with C6-HHL, was used as a negative control to see if the medium itself induce lactolysis. The test was conducted on Petri plates with LBA (Luria Bertani with agar), supplemented with 50 g/ml kanamycin and inoculated with *Chromobacterium violaceum* CV026 biosensor strain, through uniform spreading of a quantity of 250 µl of a12 hours culture. On the surface of plate inoculated in this manner were made 5 mm wells, which have been treated with 100 µl of sterile supernatant of B50 strain on LB C6-HHL. Plate were incubated overnight at 37°C and then analyzed for the presence of purple halos. The absence of halos indicated that all C6-HHL from growing medium was degraded by B50. The results have revealed that strain B50 produce lactonase.

B50 strain was tested regarding migration mobility on the surface of agar media *(swimming)* and for aggregation *(swarming).* As control was used a standard strain, genetically modified to be immobile, *P. putida* PCL1760 (Validov et al., 2007, J. Appl. Microbiol., 102: 461-471). In order to perform these tests, the strains were refreshed on LB media, supplemented with 1.8% agar, by culturing overnight at 28°C. Petri plates with 25 ml of LB medium supplemented with 0.3% agar, for *swimming,* and by 0.5% agar, for *swarming,* were prepared and allowed to dry for 20-30 minutes in the laminar-flow hood before use. The strains were inoculated by streaking the sterile media with a sterile wood toothpicks. After 18 hours of incubation at 28°C, the plates were analyzed regarding mobility. The results revealed that strain B50 presented both types of mobility.

B50 strain was tested *in vitro* regarding the formation of the biofilm by cultivation in medium CM (Fall et al., 2004, System. Appl Microbiol., 27:372-379) and the medium M63 (O'Toole and Kolter, 1998, Mol. Microbiol., 28:449-461). Together with the B50 strain were tested also two standard strains, *Pseudomonas fluorescens* WCS 365 (Simons et al., 1996, MOL. Plant-Microbe Interact., 9: 600-607) as a positive control, and *Bacillus subtilis* B168 (ATCC 23857), known as being a weak biofilm producer.

The test consisted in refreshing bacteria cells on LB media, by incubation at 28°C for 18 hours and using 10 µl from this refreshed culture for the inoculation of 0.5 ml medium CM and, respectively, M63, distributed in polypropylene sterile Eppendorf tubes. The inoculated tubes were incubated overnight at 37°C without shaking. Phenotype analysis of biofilm was done by staining adherent cells with 1% w/v Crystal violet, for 10-15 minutes, after removal of culture medium from tubes, and repeated rinsing with sterile distilled water. The biofilm formation was quantified by adding 2x200 ml 95% ethanol and washing of the adherent cells stained with Crystal violet. Ethanol solutions resulted from washing step were transferred to a 1.5 ml Eppendorf tube, adjusted to a final volume of 1 ml with sterile distilled water, and the absorbance at 540 nm was determined. Each test was done in 8 repetitions, the experiments being repeated three times, and the results were processed by analysis of variance (Statistica 10, StatSoft, Tulsa, OK, USA).

The results, presented in table 5, shows that B50 strain has the capacity to form biofilm *in vitro,* in the situation of growing on the two types of culture media tested. On both culture media, quantitative analysis of biofilm did not reveal a significant difference between the positive control, strain WCS365, on which the capacity to form abundant biofilm was associated with the activity of rhizosphere colonization (Bloemberg et al., 2000, Mol. Plant-Microbe Interact. 11: 1170-1176) and which interact with fungi producing mycorrhiza (Biancitto et al., 1996, Protoplasma, 193: 123-131), and strain described by this invention, *Brevibacillus parabrevis* B50. On the culture medium CM was observed a better development of biofilm comparing to medium M63, for all strains studied. Also the strain *Bacillus subtilis* B168, known as being a weak biofilm producer, formed a higher quantity of biofilm on the medium CM, this culture medium being designed especially to promote the formation of biofilm by the Gram-positive endo-sporing bacteria from the *Bacillales* Order.

**Tab. 5. Quantification of the biofilm formed after 24 h of culture in static conditions, at 28°C, of B50 strain and standard strains, on CM and M63 media*.**

| Strain code | Absorbance 540 nm - culture on CM media | Absorbance 540 nm - culture on M63 media |
|---|---|---|
| B50 | 0.675 ± 0.082a | 0.202 ± 0.046c |
| WCS365 | 0.612 ± 0.93a | 0.226 ± 0.051c |
| B168 | 0.186 ± 0.048b | 0.096 ± 0.026d |

| | | |
|---|---|---|
| *average of the three experiments with 8 repetitions for each strain. Values followed by the same letter does not differ significantly for < P 0.05. | | |

It was tested the capacity of bacteria *B. parabrevis* B50 to oxidize manganese, because it was reported a direct correlation between development of mychorriza and the number of bacteria that oxidize manganese from plant rhizosphere (Arines et al., 1992, Mycorrhiza, 1: 127-131; Noguiera et al., 2004, J. Plant Nutr. 27: 141-156). Bacteria were grown on agarized MPSV medium, with the following composition: 10 mM HEPES buffer, 2 g/l peptone, yeast extract 5 g/l, MnSO₄.4H₂O 0.2 g/l, FeSO₄ 0,001 g/l, agar 20 g/l, pH 7. To demonstrate the capacity of B50 to oxidize manganese to manganese dioxide was done the test with Leucoberbelin blue, acid 2-[bis (2-dimeilaminofenil) methyl] benzensulfonic, substance that forms a specific color with species of Mn (IV). In order to make this test, over the colony of bacteria developed on MPSV agarized culture medium were added a few drops of a solution 0.04% Leucoberbelin blue. After a few minutes appeared a blue coloration, which indicates accumulation of Mn (IV), formed by the oxidation of Mn (II) species by bacteria B50.

Polyamines formation by the bacteria *B. parabrevis* B50 was determined. Polyamines were reported to be involved in plant roots development (Couée et al., 2004, Plant Cell. Tiss. Organ. 76: 1-10) and stimulates the formation of mycorrhiza on plant roots (El Ghachtouli et al., 1995, Mychorriza, 5: 189-192; Wu et al., 2010, Not. Bot. Hort. Agrobot. Cluj, 38: 25-31; Cheng et al. 2012 World J. Microbiol. Biotechnol., 28:1615-1621). Bacteria were grown on a minimal medium with the following composition (g/l): glucose 10 g; L-alanine 890 mg (10 mM); L-glutamic acid 1470 mg (10 mM); L-asparagine 1320 mg (10 mM); L-lysine 1460 mg (10 mM); L-arginine 1740 mg (10 mM), mineral salts mixture 100 ml. The mixture of mineral salts used was made by dissolving, in 1 liter of water, of K₂HPO₄, 30 g; KH₂PO₄, 10 g; NH₄Cl, 5 g; NH₄NO₃, 1 g; Na₂SO₄, 1 g; MgSO₄.7H₂O, 100 mg; MnSO₄. 4 H₂O, 10 mg; FeSO₄.7H₂O, 10 mg; CaCl₂, 5 mg, and adjustment of pH to a pH 6.8-7.0 with 1 M phosphoric acid. There were prepared separately solutions of: 25 g/l glucose, amino acid mixture 25 mM in one liter, liquid mixture of mineral salts. These were sterilized separately, each of these solutions at 121°C for 20 min and then there were reunited, aseptically, 400 ml of glucose solution 25 g/l, 400 ml of the mixed solution of amino acids 25 mM/l, 100 ml of mineral salts. The pH was adjusted to 6.8-7.0 with phosphoric acid 1 M and the medium was completed to 1 liter with sterile water. The medium was then distributed in 500 ml flasks, on each 100 ml, inoculated and incubated for about 24 hours on an orbital shaker, until achieving an optical density at 600 nm OD₆₀₀ = 1.22, corresponding to approx. 1.18...1,20x10⁸ cfu/ml, which represents the end of the exponential growth phase. After reaching this threshold the bacterial culture was separated by centrifugation at 5000xg for 20 min at 4°C. The supernatant was acidified with perchloric acid, till a final concentration of 5%. The acidified supernatant was derivatized by dansylation (Cassan et al., 2009. Eur. J. Soil Biol., 45:12-19). Polyamines were separated and identified by thin-layer chromatography, using mixtures of chloroform-triethanolamine (9:1) and n-hexane-ethyl acetate (2:1) and comparing Rf values with dansylated standard compounds values. Silica gel plates were observed on UV light and selected spots were quantitatively transferred into a test tube. Dansylated polyamines were eluted from silica gel with methanol-toluene solution (9:1). Fluorescence was measured by spectrofluorimetry, using 415 nm light excitement and 510 nm light emission. Each experiment included five flasks, and the experiments were repeated three times. Strain *B. parabrevis* B50 produce 4.25±0.63 nmol/ml cadaverine in culture medium, and 5.12±0.37 nmol/ml putresceine. Cadaverine production in a synthetic culture medium is higher than that reported for the strain of *Azospirillum brasiliense* Az39, on which the ability to produce cadaverine was associated with plant growth promoting activity (Cassan et al., 2009. Eur. J. Soil Biol., 45:12-19).

Strain *B. parabrevis* B50 was analyzed in terms of innocuity on the larvae of *Galleria mellonella,* in accordance with the protocol described by Seed and Denis, 2008, Infect. Immunit. 76: 1267-1275. In order to perform this, the strain of B50 B *parabrevis* was cultivated on medium LB at 28°C, with agitation, 150 rpm for 48 hours, after which it was centrifuged at 4000 rpm, and the sediment was resuspended in 10 mm of MgSO₄, diluted and normalized, and injected into larvae of *Galleria mellonella.* Larvae have been reared on a culture substrate, at 30°C, the substrate containing: cornmeal, 400 g, wheat flour 200 g, wheat bran 200 g. All these components have been kept in the freezer for 7-10 days and after that these were sterilized by maintaining at 70°C for 2 hours. To these sterilized components were added 100 g ground dry yeast and 200 g powdered milk. To this powdery mixture were added 700 ml of liquid mixture, composed of 350 ml honey and 350 ml glycerol with suspended bees wax (1:1 w/v). The final composition was homogenized in the mixer and was kept in a plastic box, at 4°C.

The larvae of the third age were kept at 4°C above the rearing substrate mentioned above. For infection, it was used a Hamilton syringe of 5 µl. 5 µl of bacterial suspension were injected through the left side of the last segment. After the injection, the larvae were placed in the incubator, in the dark, at 30 ° C, the optimal temperature for their growth and development. Serial dilutions were made, and the larvae of *G. mellonella* were injected with a bacterial concentration of 10⁶ cfu/ml in solution 10 mM MgSO₄. The experiment was done with a control treatment, on which larvae were injected with 5 µl 10 mM solution MgSO₄ + 1.2 mg/ml ampicillin, to assess any potentially lethal effect of the injection procedure. Each experimental treatment included 60 larvae, being organized on three repetitions, each of these with lots of 20 larvae. Monitoring of larvae (dead, alive, chrysalides) was done at 48 ... 72 hours after infection, larvae being maintained at 30°C. The results are presented in table 6 from below.

**Tab. 6. Mortality Galleria mellonella larvae of in the experiment of testing innocuity of B. parabrevis B50 strain.**

| Treatment | Repetition | 24 hours after treatment | | 48 hours after treatment | | 72 hours after treatment | | |
|---|---|---|---|---|---|---|---|---|
| | | alive | death | alive | death | alive | death | chrysalides |
| Control, 10 mM MgSO₄ + 1.2 mg/ml ampicillin | R1 | 20 | 0 | 19 | 1 | 17 | 1 | 2 |
| | R2 | 20 | 0 | 20 | 0 | 18 | 1 | 1 |
| | R3 | 20 | 0 | 19 | 1 | 18 | 1 | - |
| B50 10⁶ cfu/ml in 10 mM MgSO₄ | R1 | 20 | 0 | 19 | 1 | 19 | 1 | - |
| | R2 | 19 | 1 | 18 | 2 | 17 | 2 | 1 |
| | R3 | 19 | 1 | 19 | 1 | 18 | 1 | 1 |

Data from tab. 6 demonstrates that the strain, *B. parabrevis* B50, practically do not present pathogenicity for the larvae of *Galleria mellonella,* not-having the ability to multiply in the larvae body and produce bacteremia, although were injected high concentrations into the larvae lymph fluid. The strain B50 is not pathogenic for metazoa organisms and do not present a risk for human health.

Wheat straws, sterilized by gamma irradiation, were used to determine the ability to colonize the plant material. Sterile straws, about 6 ... 7 cm, were aseptically lodged on petri plates with a diameter of 9 cm, which contained filter paper moisten with sterile phosphate buffer saline (PBS). The ends from one side of the wheat straw were inoculated with 10 µl bacterial suspension, containing 10⁶ ufc/ml B50. After 48 hours straw ends (approx.2 cm), from inoculation opposite side, were aseptically taken and put into a test tube with 10 ml of sterile PBS. From the shaken supernatant were taken 0.5 ml, which were diluted serially three times, and then inoculated on nutrient agar. The resulted colonies were re-identified as *Brevibacillus parabrevis,* based on phenotypic microarray tests on Biolog plates and API 20E tests. The conclusion of the experiment was that the bacterial strain B50 presents capacity of plant material colonization.

An experience under controlled conditions was done in order to verify the activity of protection of maize seedling against the attack of *Fusarium graminearum.* Maize seed (cv. Fundulea 376), chemically sterilized through repeated washing with hypochlorite, were bacterized with a bacterial suspension of 10⁸ cfu/ml. The experiment was done with a non-inoculated control and with a standard, treated with a chemical product (2 g/kg, metiltiofanat 70%). The infection was done by treating nutrient solution with a concentrated suspension of fungal spores, the number of spores per ml of nutrient solution being 10⁴ cfu/ml. Experimental treatments were: V₁ - inoculation *B. parabrevis* B50; V₂ -inoculation *B. parabrevis* B50 + *F. graminearum* DSM4527; V₃ - metiltiofanat 70% equiv. 2 g/kg + *F. graminearum* DSM4527; V₄ - Control not-inoculated with bacteria or fungal phytopathogen. Each treatment was carried out in 5 repetitions. Sterile growth pouches cyg (Mega International, West St. Paul, MN, USA) were used, and on each pouch were placed 3 pre-germinated seeds. The pouches were placed randomly in a growth chamber. Seedling were grown under controlled conditions, in a climatic chamber (Model GSC 120 LED, Weiss Gallenkamp, Lougborough, United Kingdom), at a temperature of 22°C±0.2°C, photoperiod 12 hours per day, light intensity of 250 µmol photons m⁻²s⁻¹, for 10 days. Final results demonstrated that B50 strain has an efficacy of 91%, with almost 3% more than chemical standard metiltiofanat 70%. In conclusion the strain B50 presents a significant antagonism for *F. graminearum* also *in vivo,* in controlled conditions.

Parts of seedling resulted from pre-germinated seeds treated with strain B50 were aseptically collected. Seedling epicotyls were passed in a test tube with 10 ml of sterile phosphate buffer saline. From the shaken supernatant were taken 0.5 ml, which were diluted serially three times and inoculated on nutrient agar. The colonies formed were re-identified as *Brevibacillus parabrevis,* based on phenotypic microarray tests on Biolog plates and API 20E tests. It was concluded that B50 strain has the ability for endophytic colonization of maize plants and for protecting these from the attack of a major phytopathogen.

*Example* 2. Strain *B. parabrevis* B50 was tested in terms of its antagonistic capacity *in vivo,* for protection of ornamental plants against phytopathogen agents from soil. Seeds of hybrid geranium *(Pelargonium x hortorum*)*,* "Moulin Rouge", were sown on April 18, to get stock plants, from which were obtained by cutting, in early September, the plants for experimental treatments. The cuttings were place in a mist chamber, in vermiculite, to stimulate roots development. After three weeks, uniform cuttings, in terms of root and shoot development, were planted in plastic pots of 15 cm, containing a growth substrate enriched with nutrients for the first weeks of growth (Canna Terra Professional Plus, Canna International BV, Oosterhout, Netherlands). Flower pots were kept in greenhouse conditions at 22±2°C during the day and 17± 2°C during the night, with a 12 hours photoperiod, supplemented with light intensity of 160 mcE/m²/s, provided by halide lamps, when light intensity decrease below 500 mcE/m²/s. Substrate contained an initial nutrient reserve and, consequently, the plants were fertilized only after three weeks of growth by applying 100 ml of nutrient solution 1 g/l of fertilizer 20-8-20 (N-P₂O₅-K₂O Eurofertil, TimacAgro, Romania).

This *in vitro* testing experiment included the following treatments.
V₁ - control, not-inoculated (with phytopathogens or B50);
V₂ - inoculated with *B. parabrevis* B50, 10⁶ spores/ml, at 4 weeks after transplantation;
V₃ -inoculated with P. *ultimum,* DSM 62987, 10⁶ propagule/ml, 8 weeks after transplantation;
V₄ -inoculated with *B. parabrevis* B50, 10⁸ spores/ml, at 4 weeks after transplanting and *Pythium ultimum,* DSM 62987, 10⁶ propagules/ml, 8 weeks after transplantation.

Each treatment included 12 pots, which were arranged in blocks of three per each repetition, in a Latin square random schedule, 4 variations in 4 repetitions.

At 4 weeks after cuttings transplantation, the soil from the treatments V₂ and V₄ was inoculated with 50 ml of sterile phosphate buffer saline, 0.1 M, pH 7.2, containing 10⁸ cfu/ml of *B. parabrevis* B50. Antagonistic strain was grown on liquid medium LB for 2 days. Biomass formed was recovered in sterile phosphate buffer, 0.1 M, pH 7.2. Normalization of the number of bacteria was done by using the colony-forming units technique. The others treatments, V₁ and V₃, were treated with 50 ml of sterile phosphate buffer, 0.1 M, pH 7.2.

At 8 weeks after cuttings transplantation were inoculated treatment V₃ and V₄ with *Pythium ultimum.* Because at the room temperature *P. ultimum* do not produce zoospores (van der Plaats-Niterink, 1981, Monograph of the genus Pythium. Institute of the Royal Netherlands Academy of Sciences and Letters, Baarn, Netherlands, pp. 164-167), the suspension of propagules of *P. ultimum* was obtained from hyphae and oospores, being prepared by cultivation on liquid medium (250 ml erlenmeyer flasks) which contained 100 ml of potato dextrose liquid medium, incubated on a rotary shaker, at 150 rpm, for 7 days at 24°C. Propagules number was determined by using a microscopic counting chamber, being brought to 10⁶ cfu/ml in a potato glucose liquid medium.

At 4 weeks after the infection with *P. ultimum,* and 12 weeks after transplantation, the fresh mass and the dry mass of roots and aerial parts (shoots and leaves) of geranium plant were weighed. The data were statistically processed by analysis of variance (Statistica, StatSoft).

The results are presented in table 7 and demonstrates both the existence of an antagonism of strain *Brevibacillus parabrevis* B50 against *P. ultimun,* as well as the capacity of this strain to determine the stimulation of growth of geranium plant, in the given experimental conditions.

**Tab. 7. Effect of treatments with B. parabrevis B50 on the development of the disease caused by P. ultimum on geraniums and on geranium plant growth.**

| Experimental treatment | root mass, g | | Shoot mass, g | |
|---|---|---|---|---|
| | fresh | dry | fresh | dry |
| V₁ -control, not-inoculate | 13.22b | 1.81 b | 109.26c | 15.12bc |
| V₂ - *B. parabrevis B50,* 10⁸ spores/ml | 16.42a | 2.94a | 129.12a | 17.86a |
| V₃ - *P. ultimum,* DSM 62987, 10⁶ propagules /ml | 10.42 c | 1.18c | 103.42 c | 14.09c |
| V₄ - *B. parabrevis B50,* 10⁸ spores/ml *P. ultimum,* DSM 62987, 10⁶ propagule/ml | 14.12ab | 2.38a | 118.73b | 16.47ab |
| DL 5% | 1.68 | 0.45 | 9.42 | 1.14 |

*Example 3.* Strain *B. parabrevis* B50 was multiplied and included in a composition which should promote its colonization of the growing substrate and/or of rhizosphere. Multiplication was done on a medium containing 10 g/l glucose, 1.5 g/l autolysed yeast, 1.0 g/l K₂HPO₄, 0.2 g/l MgSO₄*x*7H₂O, 0.1 g/l CaCl₂*x*2H₂O. pH of the liquid medium was adjusted to 6.5 with 1 N NaOH, distributed by 150 ml to 1 l erlenmeyer flasks, inoculated and agitated at 150 rpm on orbital shaker (Unimax 1010, Heidolph Instruments) at 28°C, 48 hours. After 48 hours the bacterial population reached a level of 5.8x10⁹ cfu/ml. This bacterial suspension was used for formulation as granules. 35 ml of suspension were added over 90 g of mixture consisting of 75 g oatmeal flour, 10 g mixture which includes ethyl esters of fatty acids, lecithin, potassium soaps, glycerol, lipids of rapeseed oil, and 4 g polyvinyl alcohol (26-88, powder, EMPROVE ®, Merck, Darmstadt, Germany). The resulting paste was extruded on a pasta machine (Model TR95A, Helco, Craiova, Romania), and the resulting noodles were granulated on a spheronization equipment (model Spheronis-250 m Grabler, Ettlingen, Germany). The granules were dried in a dryer in fluid bed (TC20 Model, Retsch, Germany), at a maximum temperature of 40°C.

The mixture of ethyl esters of fatty acids, lecithin, potassium soap, glycerin, unsaponifiable lipids from rapeseed oil, was obtained according to the procedure described below. 1 000 g of degummed rapeseed oil, with the characteristics shown in table 8, was brought-up in a 2 liters stainless steel autoclave, with stirring and heating, under protective atmosphere of nitrogen.

**Tab. 8. Characteristics of degummed rapeseed oil used**

| | | |
|---|---|---|
| Water and volatile compounds | % m/m | 0,4 |
| Unsaponifiable substances | % m/m | 1.4 |
| Free fatty acids | % m/m | 1.9 |
| Saponification number | mg KOH/g | 169,5 |
| The average fatty acid composition (% w/w): C16: 2.4; C18: 2; C18-1: 10.0; C18-2: 24.5; C18-3: 7.3; C20-1: 4.5; C22-1: 42.4 | | |

25 g of potassium hydroxide purity 98% were dissolved on 210 g of ethanol with 0.3% water, and the resulting solution was added in autoclaves over degummed rapeseed oil. It was started the agitation and the heating to 40°C. After a reaction time of 8 hours, the reaction mass was cooled to room temperature. 1225 g transparent reaction mass (R1) were collected. 500 g of R1 were treated with technical oleic acid, yielding a product (P1) having the following composition: (% w/w): fatty acid ethyl esters (FAEE) 74.5; not-saponified lipids 5.9; glycerol 7.1; potassium soap 11.4 and water 1.1. 140 g of the product of the reaction (P1) was treated under vigorous shaking with 60 g soy lecithin liquid emulsifiant, modified, with a hydrophilic-lipophilic balance HLB higher than 8 (Thermolec® WFC, Archer Daniels Midland, Decatur, IL, USA), resulting a mixture with the following composition: (% w/w): fatty acid ethyl esters (FAEE) 48.7; not reacted fats from rapeseed oil 4; glycerol 4.5; potassium soap 7.5, lecithin 34.6 and water 0.7. This mixture was the one from which had been taken 10 g and were used for the controlled-release composition containing strain B50.

Controlled-release formulation based on strain *B. parabrevis* B50 obtained on this manner is made up of 76 parts oatmeal flour, 3 parts ethyl esters of fatty acids, 4 parts polyvinyl alcohol, 3.5 parts lecithin, 0.75 parts potassium soaps, 0.45 parts glycerol, 0.4 parts fats from rapeseed oil, the rest of up to 100 parts water, and min. 10⁸ cfu/g *B. parabrevis* B50.

The number of bacteria *B. parabrevis* B50 in composition was checked by suspending 0.1 g of composition in sterile phosphate buffer saline, serial dilution of the initial suspension and a cultivation on nutrient agar.

*Example 4.* Strain *B. parabrevis* B50 and composition based on it, obtained according Ex. 3, were used to make mycorrhizal compost. Multiplication of mycorrhizal biotrophic fungi was done on the roots of maize and beans. The plants were growing on a growing substrate composed of soil, sand, vermiculite, and phosphate rock, in the ratio 2: 1: 1: 0, distributed in wooden boxes, lined with black polyethylene foil, perforated to allow drainage. Each box was filled with 200 liters of growth substrate, which occupied about 80% of the available volumes. Grains of maize and beans were alternatively sown, 10 cm from each other. Spores of *Glomus intraradices* MUCL 43204, from an axenic culture, have been multiply on the roots of *Medicago truncatula,* according to the protocol described by Chabaud et al. 2006, in Mathesius, U., Journet, E.P. and Sumner, L.W. (eds.), The Medicago truncatula handbook, http://www.noble.org/MedicagoHandbook, ISBN 0-9754303-1-9). Mycorrhiza fungal spores were recovered from the growth substrate through wet orbital sieving. Portion of 300 spores were disinfected through successive washing, initial disinfection for 15 min with 30 ml chloramine-T/Tween 20 solution (1 g of chloramine-T and 0.25 ml Tween 20, brought to 100 ml solution with distilled water), at room temperature, followed by an immersion on 30 ml of solution 200 mg/ml streptomycin sulfate (sterilized by ultrafiltration), for 1 h at 4°C, and final, abundant, 5 rinses with sterile distilled water.

The experiment was done with the following treatments:
V₁ - control, growth substrate not inoculated with mycorrhiza fungi or bacteria;
V₂ - growth substrate inoculated with spores of *Glomus intraradices* MUCL 43204, 300 spores per seed;
V₃ - growth substrate inoculated with spores of *Glomus intraradices* MUCL 43204, 300 per seed-spore and bacteria *B. parabrevis* B50, 10 ml x 10⁸ cfu/ml;
V₄ - growth substrate inoculated with spores of *Glomus intraradices* MUCL 43204, 300 spores per seed, and 10 g of preparation according Ex. 3, 10⁸ cfu/g B. *parabrevis* B50.

Each treatment was carried out in 4 repetitions, each repetition consisting of three boxes. The experiment was a done in randomized Latin square. Boxes with plants on which were developed mycorrhizal fungi were maintained in greenhouse conditions, at 22±2°C during the day and 17±2°C during the night, with 12 hours photoperiod, supplemented with light intensity of 160 mcE/m²/s, resulted from halide lamps when light intensity decrease below 500 mcE/m². Plants were grown for 12 weeks, being fertilized every two weeks, through the application of 100 ml of nutrient solution, 1 g/I of fertilizer 20-0- 20 (N-P₂O₅-K₂O), made from ammonium nitrate and potassium nitrate, on each box. Twice per week the plants were watered to 80% of the capacity of water retention in the growth substrate. After 12 weeks the plants were cut at the base of the stem and watering was stopped, to stimulate the production of spores of mycorrhizal fungi. After 10 days plant were extracted from the soil, roots that were cut into pieces of approx. 1 cm and then mixed back with the growth substrate. The resulted material is mycorrhizal compost, supplemented, in the case of using bacteria *B. parabrevis* B50, also with these bacteria.

In samples taken from the materials resulted on each box were determined the number of mycorrhizal fungi (AMF). The AMF number was determined by direct counting of sporocarps and spores formed in the substrate, separated by wet sieving and sucrose gradient density centrifugation (Oehl et al. 2003, Appl. Environ. Microbiol. 69: 2816-2824). The determination was done on 50 g substrate, air- dried, which was wet sieved on an orbital sieving system (model AS 200, Retsch, Haan, Germany) with 1,000-, 500-, 125-, and 32-µm sieves. Material retained on sieves of 1000-µm was checked for adjacent spores on root system, and that from 500-µm sieve was checked for spores of large dimensions, spores clusters or sporocarps. Contents of sieve 125- and 32-µm was layered on top of a solution of 70% water-sucrose (mass/volume) and centrifuged at 900 x g for 2 min. Resulted supernatant was re-passed on 32-mm sieve, washed with tap water and transferred to Petri plates. Spores, spores clusters and sporocarps, obtained from different types of sieves, were counted using a dissecting microscope at a magnification of up to x90. Then, from 50 to 70% of the spores were mounted on slides with polyvinyl alcohol, lactic acid, glycerol (Koske and Tessier, 1983, Mycol. Soc. Am. Newsl. 34: 59). Only spores which were healthy were mounted on slides. Spores were examined at a stereomicroscope (SZX10, Olympus, Tokyo, Japan) at a magnification of up to x 80. Spores abundance was determined for each sample and expressed as number of AMF spores per 50 grams of dried plant growth substrate. The data were statistically processed by analysis of variance (Statistica, StatSoft).

The results are presented in table 9. These results indicate a stimulation of the development of mycorrhiza and of the formation of mycorrhiza spores on the plants treated with spores of *Glomus intraradices* MUCL 43204, which is significantly influenced by the concomitant treatment with bacteria *B. parabrevis* B50, in the experimental treatments wherein were applied also these bacteria.

**Tab. 9. The production of endo-mycorrhizal fungal spores (AMF), per 50 grams of substrate, in the treatments from the experiment***

| Experimental treatment | AMF spore numbers | % of the treatment inoculated only with *G. intraradices* |
|---|---|---|
| V₁ - control, not-inoculated with mycorrhizal fungi or bacteria | 12± 5 | - |
| V₂ -inoculated with spores of *Glomus intraradices* MUCL 43204, 300 spores per seed | 236± 28 | 100 |
| V₃ -inoculated with spores of *Glomus intraradices* MUCL 43204, 300 spores per seed, and bacteria *B. parabrevis* B50, 10 ml x 10⁸ cfu/ml; | 314± 35 | 133.05 |
| V₄ -inoculated with spores of *Glomus intraradices* MUCL 43204, 300 spores per seed, and 10 g preparation according Ex. 3, 10⁸ cfu/g B. *parabrevis* B50. | 386± 28 | 163.55 |

| | | |
|---|---|---|
| * values which are followed by the same letter does not differ significantly for < P 0.05. | | |

The inclusion of the bacteria *B. parabrevis* B50 in the composition done in accordance with Ex. 3, of this invention embodiment, determine an additional stimulation of mycorrhiza formation, which is resulted from the better bacteria colonization of the plant growing substrate, assured by this composition.

*Example 5.* Mycorrhizal compost materials, resulted according to Ex. 4, were tested in terms of the capacity to protect seedlings of ornamental plants against the damping-off produced by *Rhizoctonia solanii,* in an experiment done in greenhouse conditions.

The inoculum of *Rhizoctonia solanii ATCC* 66873, anastomosis group AG4, was grown on wheat bran for 10 days. Wheat bran were sieved to get fraction of 0.25 ... 0.5 mm, washed with distilled water, divided into Roux flasks and sterilized, through autoclavation at 121°C for 30 min, repeated twice. Bran were distributed as 100 g in 450 ml Roux flask, moisten with 50 ml of sterile phosphate buffer, 0.1M, pH 7.2 and inoculated with 1 ml suspension 10⁶ propagules/ml, obtained from cultures on agarized media.

The growth substrate partially used for ornamental plants was represented by Potground H70 substrate (Klasmann-Delimann, Geeste, Germany), pH 6.0, which contains 25% white peat blonde, 75% dark peat and 1.5 g/I complex NPK fertilizer, designed for the production of seedlings of vegetables and flowers.

The growth of seedlings of ornamental flowers was done in plastic punnets with 32 trays (model 32, Marcoser, Matca, Galati, Romania), which had a volume of 102 ml for every tray.

Ornamental plants used for the experiment were pansies *(Viola x wittrockiana* cv. Bern), petunia *(Petunia x hybrida* cv. Colour parade F1), cockscomb *(Celosia cristata,* cv. Orange).

Potground H70 substrate was mixed with bran infected, in ratio of 0.25% v/v; 7 ml of this inoculated mixture was put at the bottom of each seedling tray, and over this substrate infected with *Rhizoctonia solanii* were deposited 68 ml mycorrhizal compost obtained in accordance with Ex. 4, with or without bacteria B. *parabrevis* B50. In the case of the non-inoculated control the growth substrate was mixed with wheat bran, sterilized by autoclavation and moist with sterile phosphate buffer, 0.1 M, pH 7.2, and growing substrate used in Ex.4 was added over this.

Experimental treatments included a treatment with a chemical standard product, formulated propamocarb (Previcur 607 SL, Bayer Crop Science, 607 g active ingredient per liter). Formulated propamocarb was 1% diluted in deionized water, and from this solution were applied 3 ml per each tray of 6.2 cm diameter, corresponding to a recommended dose of 10 ml of formulated product per square meter of soil seedlings. No-inoculated control and the treatment untreated with products against damping-off were treated with 3.25 ml sterile phosphate buffer, 0.1 M, pH 7.2.

On each tray were introduced seeds of the ornamental plants, already presented. Seedlings were maintained in the greenhouse conditions, at 22 ± 2°C during the day and 17±2°C during the night, with 12 hours photoperiod, supplemented with light of 160 mcE/m²/s intensity, produced by halide lamps, when light intensity decreased below 500 mcE/m². Growing substrate contained an initial nutrient reserves, thus the plants were fertilized only after one week of growth, by applying a 100 ml of nutrient solution, 1 g/l of fertilizer 20-8-20 (N-P₂O₅-K₂O, Eurofertil, TimacAgro, Romania).

Each treatment, V₁ - un-inoculated control, V₂ - treatment infested with R. *solanii* and untreated with products against dumping-off, V₃ -chemical treatment; V₄ -treatment with mycorrhizal compost obtained by inoculation of the multiplier plant growth substrate with *Glomus intraradices* MUCL 43204, V₅ -treatment with mycorrhizal compost obtained by inoculation of the multiplier plant growth substrate with *Glomus intraradices* MUCL 43204, and bacteria *B. parabrevis* B50; V₆ -treatment with mycorrhizal compost obtained by inoculation of the multiplier plant growth substrate with *Glomus intraradices* MUCL 43204, and preparation B. *parabrevis* B50 according Ex. 3, was done in 4 repetitions, each repetition being represented by a plastic punnets, with one tested ornamental plant. Plastic punnets were arranged in randomized block in greenhouse, host plants being randomized blocks, and various experimental treatments being randomized under the host plants sub-blocks.

Survival rate of seedlings of ornamentals plants was determined at 3 weeks after seed sowing. The data were statistically processed by analysis of variance (Statistica, StatSoft). The results are presented in table 10 from bellow, and show a high efficacy of bacterial strain *B. parabrevis* B50, present in the mycorrhizal composts done according to Ex. 4, regarding the protection against dumping-off produced by *R. solanii* AG-4 on seedling of ornamentals plants.

**Tab. 10. Influence of different experimental treatments on survival of seedling of ornamental plants grown on substrates inoculated with R. solanii AG-4**

| Experimental version | Average number of plants per tray with 32 wafers: | | |
|---|---|---|---|
| | *Viola x wittrockiana* | *Petunia x hybrida* | *Celosia cristata* |
| Control, un-inoculated with *R. solanii* AG-4 an untreated | 25.25 | 24.75 | 24.5 |
| inoculated with R. *solanii* AG-4 and untreated with products against dumping-off | 4.25 | 6.75 | 9.75 |
| Propamocarb, EQ. 10 ml p/f per m² | 19.25 | 20.75 | 22.75 |
| Mycorrhiza compost obtained by inoculation of the multiplier plant growth substrate with *Glomus intraradices* MUCL 43204 | 9.25 | 10.25 | 11.75 |
| Mycorrhiza compost obtained by inoculation of the multiplier plant growth substrate with *Glomus intraradices* MUCL 43204, and bacteria *B. parabrevis* B50 | 16.25 | 17.75 | 18.25 |
| Mycorrhiza compost obtained by inoculation of the multiplier plant growth substrate with *Glomus intraradices* MUCL 43204, and preparation *B. parabrevis* B50 according Ex. 3 | 18.25 | 20.25 | 21.5 |
| DL5% | 6.75 | 7.25 | 5.5 |

Mycorrhizal compost material which contain only *Glomus intraradices* presented a protective activity of plants against the attack of phytopathogen R. *solanii* AG-4, probably due to activation of the plant defence system, but this activity is under the efficacy threshold necessary for practical implementation. The presence of the bacteria *B. parabrevis* B50 in the mycorrhizal compost material increase the efficacy in controlling the attack of *R. solanii* AG-4. The use of the composition, done in accordance with Ex.3, determine a better colonization of this mycorrhizal compost material by the bacteria, ensuring an efficacy against the attack of *R. solanii* AG-4 similar to that of chemical product. Mycorrhizal compost material with bacteria B50 present the characteristics of a suppressive compost.

## Claims

1. Strain of *Brevibacillus parabrevis* B50, NCAIM (P) B 001413, **characterized in that** present superior characteristics of: *in vitro* antagonism against phytopathogen agents, *Fusarium graminearum, Sclerotinia sclerotiorum, Alternaria spp., Rhizoctonia solani, Fusarium oxysporum* f. sp. *dianthi , Sclerotium bataticola, Botrytis cinerea*; biosynthesis of compounds which stimulate the formation of mycorrhiza, manganese dioxide, polyamines; endophyte development; innocuity on *Galleria mellonella* test; protection of maize plants, used as host plants for the multiplication of mycorrhizal fungi, and ornamental plants of geranium, pansies, petunias and cockscomb, against soil phytopathogen agents; stimulation of mycorrhiza on the host plants used for multiplication of mycorrhizal fungi, with the formation of suppressive mycorrhizal composts

2. Controlled-release composition based on strain *Brevibacillus parabrevis* B50 **characterized in that it** is made up of 76 parts oatmeal flour, 3 parts ethyl esters of fatty acids, 4 parts polyvinyl alcohol, 3.5 parts lecithin, 0.75 parts potassium soap, 0.45 parts glycerol, 0.4 parts fats from rapeseed oil, the balance to 100 parts being water, and min. 10⁸ cfu/g *B. parabrevis* B50
